# EUROPÄISCHE PATENTANMELDUNG

(11) **EP 2 388 028 A1**
(43) Veröffentlichungstag der Anmeldung: **23.11.2011**
(21) Anmeldenummer: 10075214.6
(22) Anmeldetag: 20.05.2010
(51) Int. Cl.: A61M 1/10, F04B 49/20

(54) **Verfahren zum Betrieb eines Pumpensystems**

(71) Anmelder: Berlin Heart GmbH, 12247 Berlin (DE)
(72) Erfinder: Hering, Jörg, 15370 Fredersdorf (DE); Arndt, Andreas, 12555 Berlin (DE)
(74) Vertreter: Pfenning, Meinig & Partner GbR

(57) **Zusammenfassung**

Bei einem Verfahren zum Betrieb eines Pumpensystems mit einer pulsatilen Membranblutpumpe (2) zum abwechselnden Ansaugen und Ausstoßen von Blut im Blutkreislauf eines Patienten, die mittels einer Druckleitung (3) mit einer Arbeitspumpe (4) antreibbar ist, wobei die Arbeitspumpe einen mittels eines Antriebsaggregats (24) oszillierend antreibbaren Arbeitskolben (18) aufweist, stellt sich die Aufgabe, die Arbeitspumpe effizienter als bisher anzusteuern und damit ein dynamischeres Verhalten und eine bessere Regelbarkeit der Pumpe zu erreichen.

## Beschreibung

Die Erfindung liegt auf dem Gebiet der Feinwerktechnik und der Elektrotechnik und beschäftigt sich insbesondere mit einem Steuerungsverfahren, mittels dessen ein komplexes Pumpensystem gesteuert werden soll.

Insbesondere ist die Erfindung vorteilhaft auf dem medizinischen Gebiet einsetzbar.

Es sind Pumpensysteme, insbesondere auf medizinischem Gebiet, bekannt geworden, die zur Unterstützung des Blutkreislaufs von Menschen vorteilhaft einsetzbar sind. Derartige Pumpen können im Einzelfall auch als Kunstherz das Herz eines Menschen zumindest zeitweise ersetzen.

Eine Ausführungsform eines solchen Herzens umfasst typischerweise eine Membranpumpe, die innerhalb oder außerhalb eines Körpers angeordnet sein kann und mit dem Blutkreislauf über eine Ansaugöffnung und eine Ausstoßöffnung verbunden ist. Die Pumpe ist als Membranpumpe, Beutelpumpe oder eine ähnliche Bauform mit einem Antriebsraum und einem Blutförderraum, die durch eine bewegliche Membran voneinander getrennt sind, ausgebildet. Der Antriebsraum ist mittels einer Arbeitspumpe druckbeaufschlagbar, so dass die Lage der Membran durch die Steuerung des Antriebsdruckes gezielt verändert werden kann, um in dem Blutförderraum Über- oder Unterdruck zu erzeugen und damit Blut aus dem Blutkreislauf anzusaugen oder in diesen auszustoßen. Ventilgesteuert kann damit jeweils während der Diastole Blut durch ein Ventil angesaugt und dieses während der Systole durch ein anderes Ventil ausgestoßen werden.

Die entsprechende Blutpumpe sollte derart eingerichtet sein, dass sie in ihrem Betrieb an die Herzfrequenz des Patienten, an den geforderten Blutdruck und das zu fördernde Volumen angepasst werden kann. Insbesondere ist eine Membranregelung erforderlich, die die Zufluss- und Abflussbedingungen auf der Blutseite berücksichtigend dafür sorgt, dass die Membran in beiden Richtungen gleichmäßig ausgetrieben wird.

Auch die Phasenlage der zyklischen Membranpumpentätigkeit kann an den Herzschlag des Patienten anzupassen sein.

Hierzu wird üblicherweise die Steuerung der Arbeitspumpe den Erfordernissen entsprechend gestaltet. Es ist hierbei jedoch eine komplexe, sehr schnelle und dynamische Steuerungsmöglichkeit notwendig, die eine Reaktion und ein Nachregeln möglichst innerhalb eines Zyklus der Arbeitspumpe erlaubt. Dies ist insbesondere für Blutpumpen mit kleinem Fördervolumen, beispielsweise 10 ml, wie sie zum Beispiel bei Kleinkindern eingesetzt werden, wichtig.

Der vorliegenden Erfindung liegt damit die Aufgabe zugrunde, ein Betriebsverfahren für ein Pumpensystem der eingangs genannten Art zu schaffen, das mit möglichst einfachen Mitteln eine effektive und schnell wirkende, dynamische Steuerung des Pumpensystems erlaubt.

Die Aufgabe wird durch die Erfindung mit den Merkmalen des Patentanspruchs 1 gelöst.

Die Erfindung sieht dabei ein Verfahren zum Betrieb eines Pumpensystems vor mit einer als Kolbenpumpe ausgebildeten Arbeitspumpe, die einen mittels eines Antriebsaggregats oszillierend zwischen zwei Umkehrpunkten in einem Druckraum antreibbaren Arbeitskolben aufweist, wobei die Kolbenpumpe derart angesteuert wird, dass der Antriebskolben jeweils nach Durchlaufen eines Umkehrpunktes eine Beschleunigungsphase durchläuft und danach zum Ende des vorgegebenen Weges verzögert wird.

Die Erfindung sieht zudem in einer vorteilhaften Ausgestaltung ein Verfahren zum Betrieb eines Herzpumpensystems mit einer pulsatilen Membranblutpumpe zum abwechselnden Ansaugen und Ausstoßen von Blut im Blutkreislauf eines Patienten vor, die mittels einer Druckleitung mit der Arbeitspumpe antreibbar ist, wobei jeweils nach Durchlaufen eines Umkehrpunktes vor Erreichen des Maximal- oder Minimaldrucks eine Geschwindigkeit des Arbeitskolbens der Arbeitspumpe erreicht wird, die betragsmäßig größer, insbesondere wenigstens 10%, vorteilhaft mehr als 30%, weiter vorteilhaft mehr als 50% größer ist als die Geschwindigkeit bei Erreichen des Extremaldrucks.

Durch die Erfindung wird erreicht, dass der Arbeitskolben nach Durchlaufen eines Umkehrpunktes möglichst schnell beschleunigt wird und somit in minimaler Zeit den nächsten Arbeitshub soweit durchläuft, dass ein entsprechender Druck im Druckraum aufgebaut wird. Dies ist besonders bei kleinen Pumpen vorteilhaft, deren Volumen im Verhältnis zum Volumen der Druckleitungen kleiner ist als bei großen Pumpen und mit denen ein schneller Druckaufbau im Gesamtsystem entsprechend schwieriger ist. Vorteile ergeben sich ebenfalls bei Pumpen, die mit hohen Raten betrieben werden sollen, da die die Pumpe antreibende Druckluft verzögert über den Luftschlauch auf die Blutpumpenmembran wirkt.

Eine vorteilhafte Ausgestaltung der Erfindung sieht vor, dass dem Arbeitskolben wenigstens in einem ersten, sich unmittelbar an einen Umkehrpunkt anschließenden Wegbereich ohne eine Geschwindigkeitsbegrenzung Energie zugeführt wird.

Diese Maßnahme kann beispielsweise bedeuten, dass das Antriebsaggregat keine Steuerung/Regelung der Geschwindigkeit enthält, im Falle eines rotierenden Antriebsmotors entsprechend keine Drehzahlregelung und keine Drehzahlbeschränkung. Beispielsweise kann stattdessen die Leistung des Antriebsaggregats gesteuert oder geregelt sein. Damit kann der Arbeitskolben maximal beschleunigt werden, insbesondere im ersten Teil der Bewegung eines Arbeitshubes, wenn sich im Druckraum noch kein bremsender Gegendruck aufgebaut hat.

Zudem kann vorteilhaft vorgesehen sein, dass dem Arbeitskolben wenigstens in dem ersten Wegbereich Energie durch das Antriebsaggregat und zusätzlich die in dem Arbeitsraum vor Erreichen des Umkehrpunktes durch den erzeugten Unter- oder Überdruck gespeicherte Energie zugeführt wird.

Damit wird die elastische Energie des als Gasfeder wirkenden Druckraums zur Anfangsbeschleunigung des Arbeitskolbens ausgenutzt.

Außerdem kann gemäß der Erfindung vorgesehen sein, dass die Arbeitspumpe, die über eine Druckleitung die Membranblutpumpe antreibt, einen seinerseits mittels eines Antriebsaggregats oszillierend antreibbaren Arbeitskolben aufweist, wobei das Antriebsaggregat bezüglich seines Drehmoments und/oder seiner Leistung als Zielgröße gesteuert wird.

Die durch den als Gasfeder wirkenden Druckraum auf den Arbeitskolben übertragene Energie ist schwierig bei der Steuerung zu berücksichtigen, jedoch ist insgesamt zur Erzeugung einer hohen Dynamik wichtig, zur Beschleunigung des Kolbens möglichst viele Ressourcen zu nutzen. Daher bietet sich eine Drehmoment-oder Leistungssteuerung an, so dass der Kolben in der Anfangsphase nach Durchlaufen eines Umkehrpunktes durch die Restdruckunterstützung besonders stark beschleunigt wird. Dies erlaubt den Betrieb der Pumpe auch bei hohen Raten (bis 150 Zyklen pro Minute) und großen Hüben.

Durch die Regelung der Leistung des Antriebsaggregats/ der Antriebskraft auf den Arbeitskolben ergibt sich im Zusammenspiel mit dem sich variabel einstellenden Gegendruck ein bestimmtes Geschwindigkeitsprofil des Arbeitskolbens.

Nachdem das Arbeitsvermögen des im Arbeitsraum gespeicherten Restdrucks aufgebraucht ist, wird das weitere Geschwindigkeitsprofil des Kolbens im Wesentlichen von der eingeprägten, vorgegebenen Leistung oder dem Drehmoment des Motors und im Weiteren von dem sich aufbauenden Gegendruck bestimmt.

Die mechanische Ausführung des Kolbens kann in einem innerhalb eines Zylinders dichtend gleitenden zylindrischen Kolben bestehen, oder im antreibbaren Boden eines Faltenbalgs o.ä. Der Kolbenantrieb kann mittels eines elektrischen Rotationsmotors beispielsweise durch Antrieb einer Gewindespindel mittels des Motors realisiert sein, wobei der Kolben mit einer auf der Spindel axial beweglichen, drehfest montierten Gewindemutter zusammenhängt.

Besonders einfach kann zur Steuerung des Drehmoments ein Elektromotor mittels eines eingeprägten, für einen Arbeitshub oder einen Arbeitszyklus des Antriebskolbens vorherbestimmten Stromstärkenverlaufs angesteuert werden.

Unter dem Stromstärkenverlauf wird in diesem Zusammenhang die Abhängigkeit der Stromstärke von dem zurückgelegten Kolbenweg oder dem Drehwinkel der Motorwelle oder auch von der seit der letzten Kolbenumkehr verstrichene Zeit verstanden. Eine derartige Steuerung ist in der Leistungselektronik üblich und einfach aufzubauen. Die einzuprägende Stromstärke kann auch in einem Steuerungsprozess vorgebbar sein. Sie kann während des Kolbenantriebs über einen Zyklus hinweg konstant sein oder regelmäßig einem Stromstärke/Weg oder Stromstärke/Zeit-Profil folgen.

Die Erfindung kann auch vorsehen, dass bei dem jeweils zu ermittelnden Stromstärkenverlauf Messwerte, aus denen Abweichungen der Ist- Werte vom Sollhub und der Sollrate ermittelt werden können, oder die ermittelten Abweichungen berücksichtigt werden.

Vorteilhaft kann vorgesehen sein, dass der Motor mittels eines zumindest zeitweise linearen Stromstärkenverlaufs angesteuert wird. Dieser kann beispielsweise in mindestens einer der beiden zyklisch wiederkehrenden Bewegungsphasen des Kolbens - in Systole und/oder Diastole - eine lineare Anstiegsrampe aufweisen. Dies bedeutet, dass während der Kolbenbeschleunigung, die durch die Restdruckenergie stattfindet, der Steuerstrom über die Rampe langsam erhöht wird. Dadurch werden mechanische Belastungen des Systems begrenzt.

Nach Durchlaufen der Anstiegsrampe kann die Stromstärke zum Antrieb des Elektromotors für den weiteren Verlauf der Bewegungsphase entweder konstant gehalten werden oder weiter linear ansteigen, z. B. mit einer geringeren Steigung als während der Anfangsrampe. Dies kann sinnvoll sein, da während des Durchlaufs eines Zyklus der Gegendruck steigt, der von dem Kolben zu überwinden ist, und entsprechend bei einem konstanten Stromverlauf die Drehzahl des Elektromotors sich wegen des Widerstandes verringern würde. Durch den zusätzlichen Anstieg kann das Geschwindigkeitsprofil individuell den Bedürfnissen angepasst werden.

Gemäß einer vorteilhaften Ausführungsform der Erfindung kann vorgesehen sein, dass die Bewegungsgeschwindigkeit des Motors und/oder des Kolbens, insbesondere die Drehzahl des Motors, erfasst wird. Die genannten Bewegungsgrößen sind im Wesentlichen abhängig von der Steuerstromstärke des Motors und den mechanischen Randbedingungen von Motor und Kolben, jedoch wesentlich auch von dem im Druckraum der Arbeitspumpe aufgebauten Druck zu Beginn der Kolbenbewegung bis zu ihrem Ende. Damit ergibt sich bei Erfassung der genannten Bewegungsgrößen eine Information, die zur Steuerung des Pumpensystems herangezogen werden kann. Wenn der Zeitpunkt der Messung der Bewegungsgröße/Drehzahl des Motors richtig gewählt ist, hängt diese im Wesentlichen von der Anfangsbeschleunigung des Kolbens durch den Restdruck im System oder auch nur von der Füllung des Arbeitsraums der Membranpumpe ab.

Damit kann mit der Messung von Bewegungsgrößen auf den Druck im Druckraum der Antriebspumpe bzw. die Füllung des Arbeitsraums der Membranpumpe rückgeschlossen werden.

Es kann vorteilhaft vorgesehen sein, dass die Bewegungsgeschwindigkeit des Motors und/oder des Kolbens, insbesondere die Drehzahl des Motors, ebenso wie die Lage des Arbeitskolbens, ständig erfasst wird. Diese Größen können zyklisch wenigstens einmal zu einem festen Zykluszeitpunkt während jeder Saug- und jeder Druckphase (Diastole und Systole) ausgewertet werden. Dabei kann vorgesehen sein, dass die Messung der Bewegungsgeschwindigkeit des Motors und/oder des Kolbens jeweils zu einem Zeitpunkt stattfindet, zu dem die gemessene Größe im Wesentlichen von dem durch die Arbeitspumpe erzeugten Druck abhängt.

Die Messwerte bezüglich der Position des Antriebskolbens können zur Vorherbestimmung eines Antriebsmomentverlaufs/ Stromstärkenverlaufs des Antriebsaggregats für den nächsten Zyklus der Arbeitspumpe verwendet werden. Messwerte bezüglich des Drucks können zur Steuerung eines Steuerventilserwendet werden, über das die Gasmenge im Druckraum der Arbeitspumpe sowie in den Gasleitungen eingestellt wird.

Eine vorteilhafte Umsetzung der Erfindung sieht dabei vor, dass ein Steuerventil, das den Druckraum der Arbeitspumpe mit einem Ausgleichsvolumen verbindet, zyklisch in Abhängigkeit von ermittelten Abweichungen der gemessenen Bewegungsgeschwindigkeiten oder Drehzahlen oder der gemessenen Druckverläufe hieraus abgeleiteter Druckwerte von vorgegebenen Referenzwerten angesteuert wird. Mittels des Steuerventils kann somit aus dem Arbeitsraum der Arbeitspumpe das Arbeitsgas, beispielsweise Luft, zu einem bestimmten Zeitpunkt in das Ausgleichsvolumen, beispielsweise die Umgebung der Pumpe unter Atmosphärendruck ausgestoßen werden, oder es kann in der Ansaugphase über das Steuerventil Luft angesaugt werden. Damit wird die Gesamtmenge des im System befindlichen Gases jeweils während eines Zyklus geregelt, so dass der mittlere Druck im System jeweils durch entsprechende Betätigung des Steuerventils geregelt werden kann. Dabei wird die Öffnungsdauer des Steuerventils vorteilhaft jeweils zu Anfang der jeweiligen Ansaug-oder Druckphase vorgegeben.

Es kann auch vorteilhaft vorgesehen sein, dass jeweils die Maximaldrehzahlen des Motors in der Druck-und in der Saugphase bestimmt und miteinander verglichen werden. Aus den jeweils erreichten Maximaldrehzahlen des Motors ergibt sich die erzielte Membranauslenkung der Membranpumpe. Dieser hängt unter anderem ab von der Unterstützung der Kolbenbeschleunigung durch den Restdruck im Arbeitsvolumen. Diese Messgrößen oder beispielsweise ihre Differenz können/kann als Eingangsgröße für eine Regelung des Steuerventils genutzt oder auch mit Referenzwerten verglichen werden, um eine Nachsteuerung des Steuerventils im Falle der Abweichung zu bewirken.

Es kann vorteilhaft auch vorgesehen sein, dass aus den beiden Drehzahlen, die in Systole und Diastole jeweils zu dem Zeitpunkt gemessen werden, wenn der Druck den mittleren Systemdruck erreicht hat, insbesondere aus der Differenz dieser beiden Drehzahlen, die Steuergröße für ein Steuerventil berechnet wird, das den Arbeitsraum der Arbeitspumpe für eine steuerbare Zeit während des Systole und der Diastole, insbesondere jeweils nach dem Durchlaufen des Drucknullwertes, mit einem Ausgleichsvolumen verbindet.

In Abhängigkeit von der zu fördernden Blutmenge und/oder der Herzschlagrate bzw. der angestrebten Zykluszeit des Pumpensystems und dem gewünschten Hub der Arbeitspumpe kann die anzusteuernde Leistung bzw. das Zieldrehmoment des Elektromotors bzw. der entsprechende Zeitverlauf vorgegeben werden. Diese Größe kann beispielsweise auch dadurch während des Betriebs verändert werden, dass jeweils vor einem Zyklus der Arbeitspumpe ein Stromprofil des Elektromotors insgesamt angehoben oder eine andere Steigung im Verlauf eines Zyklus gewählt wird. In der Druckphase kann der Kolben zur Minimierung des Totvolumens im Zylinder bis dicht an den Systolenendpunkt bewegt werden. Rate und Gesamthub werden durch Wahl des Stromprofils an die zur Verfügung stehende Zykluszeit und das zu fördernde Volumen angepasst.

Zur Steuerung bzw. Regelung der Leistung/Stromstärke kann bei vorgegebenem Hub als Eingangsgröße des Reglers bei Erreichen der Endposition des Kolbens eine übrig bleibende Zeitdifferenz dienen oder, wenn die Stromstärke zu gering ist, beim Ablauf der Zykluszeit der Restweg bis zur vorgegebenen und noch nicht erreichten Endposition (Umkehrpunkt) des Kolbens. Nur wenn der Kolben zur vorgegebenen Zeit genau die Endposition erreicht hat, bleibt diese Regelung des Stromstärkenprofils inaktiv.

Die Erfindung bezieht sich zudem auf ein Verfahren, bei dem während des Pumpenbetriebs ein Abbremsen der Arbeitskolbenbewegung darauf untersucht wird, an welcher Position des Kolbens diese auftritt und/ oder wie stark die Bremsbeschleunigung ist und dass bei einer bestimmten Abweichung vom Normalverhalten auf Vorliegen einer Blockade der Druckleitung oder der Blutleitungen (Kanülen) erkannt wird. Dadurch kann bei einer Verklemmung der Druckleitung oder der Kanülen automatisch ein Alarm ausgelöst werden.

Die Erfindung bezieht sich außer auf ein Verfahren der beschriebenen Art auch auf ein Pumpensystem, betreibbar mit einem derartigen Verfahren mit einer Membranblutpumpe, die durch eine Arbeitspumpe mittels einer Druckleitung antreibbar ist, wobei die Arbeitspumpe einen mittels eines Antriebsaggregats oszillatorisch antreibbaren Kolben in einem Zylinder aufweist, gekennzeichnet durch ein mittels einer Steuerung ansteuerbares Steuerventil, das einen Druckraum der Arbeitspumpe mit einem Ausgleichsvolumen verbindet.

Im Folgenden wird die Erfindung anhand eines Ausführungsbeispiels in einer Zeichnung gezeigt und anschließend beschrieben.

Dabei zeigt
- Fig. 1: schematisch die Anordnung eines Herzpumpensystems an einem Menschen,
- Fig. 2: schematisch das Zusammenwirken der Arbeitspumpe mit der Membranblutpumpe,
- Fig. 3: den Aufbau der Arbeitspumpe schematisch in einem Längsschnitt,
- Fig. 4: über einen Teil eines Bewegungszyklus die Entwicklung der Stromstärke, der Drehzahl des Motors und des Drucks in dem Arbeitsvolumen der Arbeitspumpe in einem Diagramm,
- Fig. 5: das Zusammenwirken der Arbeitspumpe mit einigen Messsensoren,
- Fig. 6: mögliche einzuprägende Stromprofile für die Ansteuerung des Elektromotors und
- Fig. 7: eine dreidimensionale Übersicht über das Pumpensystem

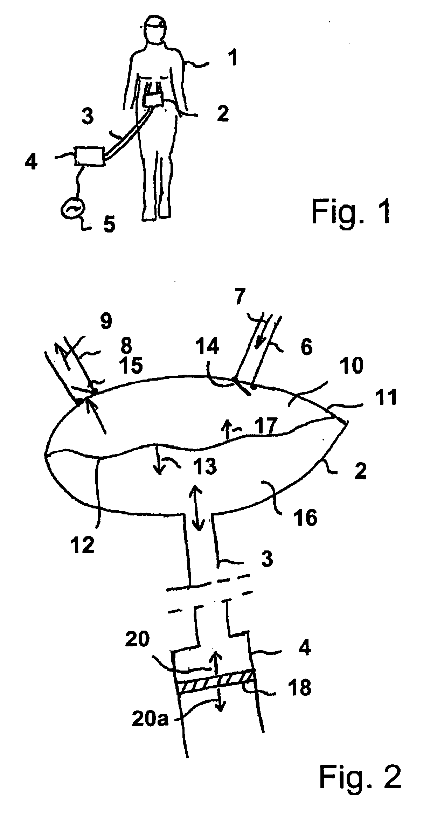

Fig. 1 zeigt schematisch eine Silhouette 1 eines Menschen, der an eine extrakorporale Membranblutpumpe 2 angeschlossen ist, die über zwei Anschlussleitungen, eine Ansaugleitung und eine Ausstoßleitung mit dem Blutkreislauf des Menschen verbunden ist.

Die Membranblutpumpe 2 ist über eine Druckleitung 3 mit einer Arbeitspumpe 4 verbunden, die die Membranpumpe 2 antreibt. Die Arbeitspumpe 4 wird über eine Energiequelle 5 elektrisch mit Energie versorgt.

Derartige Pumpsysteme können ortsfest installiert sein, sie können jedoch auch transportabel sein und beispielsweise von einem Patienten in einem Rucksack mit sich getragen werden. Die Energiequelle 5 ist dann üblicherweise eine Batterie, die den Motor der Arbeitspumpe speist.

Die Arbeitspumpe ist üblicherweise eine Kolbenpumpe, die in der Fig. 2 schematisch dargestellt und mit 4 bezeichnet ist. Sie weist einen Zylinder auf, in dem ein Kolben 18 in Richtung der Pfeile 20, 20a zwischen zwei Umkehrpunkten pendelnd bzw. oszillierend antreibbar ist. Mit dieser Pendelbewegung wird in der Druckleitung 3 abwechselnd Über- und Unterdruck erzeugt, der in dem Antriebsraum 16 der Membranpumpe 2 ebenfalls Über- und Unterdruck erzeugt. Erzeugt die Arbeitspumpe Unterdruck, so wird die Membran 12 in Richtung des Pfeils 13 gezogen, so dass in dem Arbeitsraum 10 ein Unterdruck entsteht, der zum Ansaugen von Blut über die Einströmleitung 6 in Richtung des Pfeils 7 und durch das Einwegventil 14 führt.

Der Arbeitsraum 10 wird auf diese Weise mit Blut gefüllt. Um das Blut auf zellulärer Ebene nicht zu beschädigen und auch in dem Arbeitsraum 10 keine Kavitationen zu erzeugen, muss die Strömungsgeschwindigkeit des Blutes in den Arbeitsraum 10 und aus ihm heraus in bestimmten Grenzen gehalten werden. Dies stellt besondere Anforderungen an die Beschleunigung der Membran 12 und damit an die Leistungsfähigkeit der Arbeitspumpe 4.

Ist der Arbeitsraum 10 mit Blut gefüllt, so schließt das Ventil 14. Der Kolben 18 hat dann seinen unteren Umkehrpunkt nach einer Bewegung in Richtung des Pfeils 20a erreicht und bewegt sich im Zuge einer Kompressionsbewegung nach oben in Richtung des Pfeils 20. Damit wird die Membran 12 in Richtung des Pfeils 17 bewegt, und der Arbeitsraum 10 komprimiert. Das Ventil 15 öffnet sofort oder verzögert bei einem bestimmten voreingestellten Auslösedruck und lässt Blut durch die Ausströmleitung 8 in Richtung des Pfeils 9 ausströmen. Wichtig ist, dass die Kolbenpumpe 4 in der Kompressionsphase kräftig genug ist, um die Membran bis zu ihrem Umkehrpunkt kräftig in Richtung des Pfeils 17 zu bewegen.

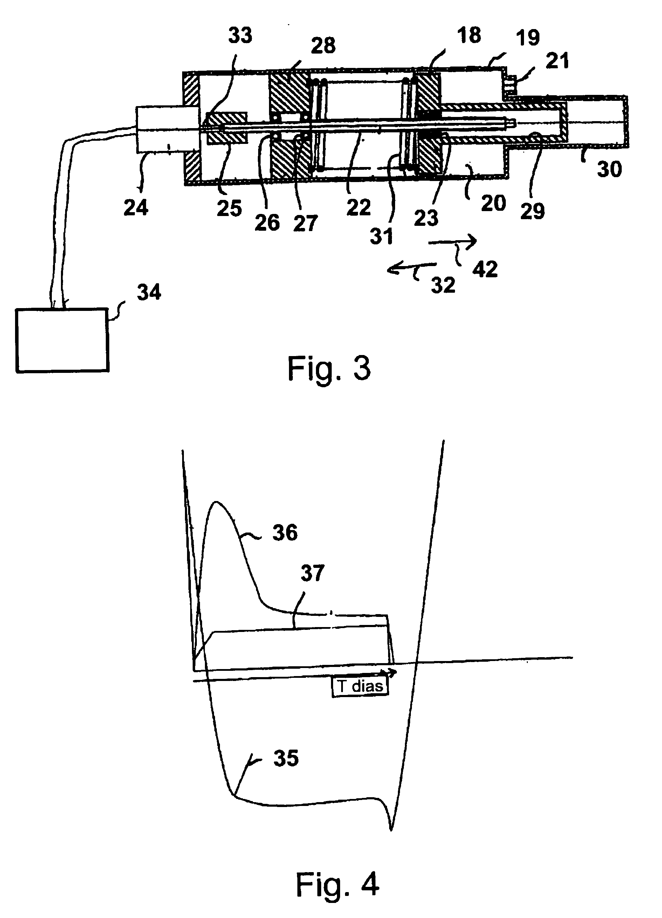

Die genauere Funktion der Arbeitspumpe 4 wird im Folgenden anhand der Fig. 3 erläutert.

In der Fig. 3 ist der Arbeitskolben mit 18 bezeichnet, der Druckraum der Arbeitspumpe 4 mit 20. Im Druckraum 20 wird das Arbeitsgas, typischerweise Luft, komprimiert, um über die Leitung 3 aus der Ausströmöffnung 21 auszuströmen. Der Zylinder trägt das Bezugszeichen 19 und ist derart eingerichtet, dass der Kolben in ihm dicht gleitet.

Der Antrieb wird durch einen Elektromotor 24 geleistet, der eine Motorwelle 33 mit einem Anschlussstück 25 antreibt. Mit dem Anschlussstück 25 ist eine Gewindespindel 22 verbunden, die in zwei Lagern 26, 27 innerhalb eines Lagerblocks 28 in dem Zylinder 19 drehbar gelagert ist.

Die Spindel wirkt mit einer Gewindemutter 23 zusammen, die in dem Kolben 18 drehfest gelagert ist. Damit bewirkt die Spindel bei einer Drehung in einer ersten Richtung einen axialen Antrieb des Kolbens in Richtung des Pfeils 42, bei der umgekehrten Drehrichtung einen Antrieb des Kolbens in Richtung des Pfeils 32.

Entsprechend komprimiert der Kolben 18 den Druckraum 20 des Zylinders 19, so dass das Arbeitsgas, insbesondere Luft, durch den Anschluss 21 in die Druckleitung 3 hineingepresst oder aus dieser herausgesaugt wird.

Der Kolben 18 ist mit einem Führungsrohr 29 gasdicht verbunden, in das die Spindel 22 hineinragt. Auf diese Weise kann die Gewindemutter 23 sich entlang der gesamten Spindel bewegen. Das Führungsrohr 29 ist optional in einer Führung 30 geführt, die es bei Erreichen des Umkehrpunktes des Kolbens in der Kompressionsphase praktisch völlig ausfüllt. Damit ist der Totraum des Zylinders 19 minimiert.

Zur Unterstützung des Kolbenantriebes kann eine Feder 31 vorgesehen sein, die während der Saugphase durch den Motorantrieb 24 über die Spindel komprimiert wird und während der Druckphase die gespeicherte Energie zur Entlastung des Antriebes an den Kolben zusätzlich abgibt. Durch diesen Mechanismus werden die Anforderungen an das Antriebssystem während der Druckphase etwas gesenkt, während sie in der Saugphase erhöht werden.

Da an das Pumpsystem während der Druckphase insgesamt höhere Anforderungen durch den hohen erforderlichen systolischen Druck gestellt werden, kann durch diese Unterstützung mittels der Feder 31 die Gesamtanforderung an den Antrieb symmetrisiert und über die Zeit ausgeglichen werden.

Zusätzlich zu den mechanischen Elementen der Arbeitspumpe 4 ist die Strom- und/oder Leistungssteuerung 34 schematisch eingezeichnet. Diese kann beispielsweise eine feste, konstante oder einer bestimmten festgelegten Kurve (in bezug auf den Kolbenweg oder den Drehwinkel der Spindel) folgende Stromstärke an den Motor 24 liefern.

Anhand der Fig. 4 soll im Folgenden die Entwicklung der Stromstärke, der Motordrehzahl und des Drucks in dem Druckraum 20 des Kolbens während einer diastolischen Phase genauer beschrieben werden.

Zur Beginn der beschriebenen Phase ist der Druck, dargestellt durch die Kurve 35, im Druckraum 20 der Arbeitspumpe 4, und damit auch in der Druckleitung 3 und im Antriebsraum 16 der Blutmembranpumpe, maximal. Der Kolben hat seinen Umkehrpunkt nach vollständiger Kompression des Volumens im Druckraum 20 erreicht, und das Blut wird aus dem Arbeitsraum 10 der Membranblutpumpe ausgestoßen. Gleichzeitig bewegt sich der Kolben zur Einleitung der Saugphase in Richtung des Pfeils 32 in Fig. 3. Der Druck 35 nimmt in dieser Phase sehr schnell ab.

Beim Umkehren der Bewegungsrichtung des Kolbens 18 muss auch der Motor 24 mit der Spindel 22 seine Drehrichtung umkehren. Aufgrund des Trägheitsmomentes von Motor und Spindel benötigt dieser Vorgang eine gewisse Zeit, und die Spindel muss nach der Drehrichtungsumkehr erst beschleunigt werden. In dieser Phase kann gegebenenfalls der eingeprägte Strom, mit dem der Motor 24 versorgt wird, in einer Rampe bezüglich der Stromstärke 37 hochgefahren werden.

Einen wesentlichen Anteil an der Beschleunigung der Spindel trägt in dieser Phase der noch in dem Druckraum 20 vorherrschende Druck, der den Kolben 18 in Richtung des Pfeils 32 zurücktreibt.

Da die Drehgeschwindigkeit der Spindel und des Motors nicht beschränkt und nicht gesteuert sind, kann diese Beschleunigung sich voll auswirken, so dass die Drehzahl der Spindel gleichzeitig mit dem abfallenden Druck 35 eine Spitze, erkennbar in der Kurve 36 der Fig. 4, aufweist. Für diese Spitze ist im Wesentlichen die mechanische Beschleunigung durch die potentielle Energie des komprimierten Gases verantwortlich, jedoch je nach der Steilheit der Rampe der Stromstärke zusätzlich der elektrische Antrieb.

Der Druck unterschreitet in dieser Phase die Nulllinie, und dies führt zu einem Beginn der Saugphase der Arbeitspumpe 4. Mit dem weiteren Abfallen des Drucks und der Erzeugung eines entsprechenden Saugdrucks trifft der Kolben 18 nun auf Gegenkräfte, die ihn zurückzuhalten versuchen, wodurch die Drehzahl 36 bei gleich bleibendem Strom reduziert wird. Der Strom ist in der Kurve 37 bezüglich der Stromstärke dargestellt. Vor einem Erreichen des Minimaldrucks erreicht die Drehzahl ein Maximum.

Ist der untere Umkehrpunkt des Kolbens erreicht, so wird die Drehrichtung des Motors wiederum umgedreht und die Spindel beschleunigt. Gleichzeitig mit der beginnenden Kompressionsbewegung des Kolbens 18 strömt noch weiter Blut durch den Einlasskanal in die Membranpumpe hinein und bewegt die Membran 12 ein letztes Stück in Richtung des Pfeils 13, so dass sich der Druck, dargestellt durch die Kurve 35 in Fig. 4, sehr steil entwickelt und schnell auf null zubewegt bzw. sich dann weiter erhöht. Darauf folgt eine Kompressionsphase, die sinngemäß ebenso zu beschreiben ist wie die beschriebene Saugphase.

Durch die beschriebene Ansteuerung des Motors mit einem vorbestimmten Stromstärkeverlauf bzw. elektrischen Leistungsverlauf wird sichergestellt, dass die in dem Gas gespeicherte Druckenergie beim Umkehren der Drehrichtung des Antriebsmotors vollständig genutzt und in eine Beschleunigung des Arbeitskolbens und der Spindel umgesetzt wird. Das System wird in dieser Phase nicht durch eine Drehzahlregelung gebremst. Dadurch ist ein sehr dynamischer Wechsel zwischen Druck- und Saugphase möglich. Im Übrigen funktioniert die Konstantstromregelung bei Funktionsstörungen wie beispielsweise Blockieren einer Kanüle wie eine Druckregelung: Sobald eine Kanüle blockiert ist, wird in der Druckphase durch die Druckerhöhung bei gleich bleibendem Drehmoment die Drehzahl der Spindel verringert, so dass sich der Blutdurchsatz verringert. Die Verringerung der Drehzahl kann überwacht und zum Nachweis einer Einschnürung einer Kanüle und für eine Alarmauslösung verwendet werden.

Der absolute Hub der Pumpe wird je nach der Pumpengröße und dem zu fördernden Volumen eingestellt. Im Zusammenspiel mit dem eingestellten Stromstärkeprofil und der erreichten Herzrate wird damit auch der absolute Durchsatz von Flüssigkeit durch die Membranpumpe bestimmt. Ansonsten passt sich durch die Regelung der Antrieb an jede zu betreibende Pumpe selbsttätig an. Ist die eingeprägte Stromstärke/Leistung zu gering, um in der vorgegebenen Zykluszeit den Kolben 18 zwischen seinen geplanten Umkehrpunkten hin- und herzubewegen, so wird dies anhand von Weg/Näherungssensoren registriert und führt zu einer erhöhten Stromstärke/Leistung beim nächsten Arbeitshub des Kolbens, bzw. wenn mittels einer Zeitmessung ermittelt wird, dass der Kolben zu schnell bewegt wird, führt dies zu einer Auswahl eines Stromprofils mit einer reduzierten Stromstärke/Leistung für den nächsten Bewegungszyklus des Kolbens.

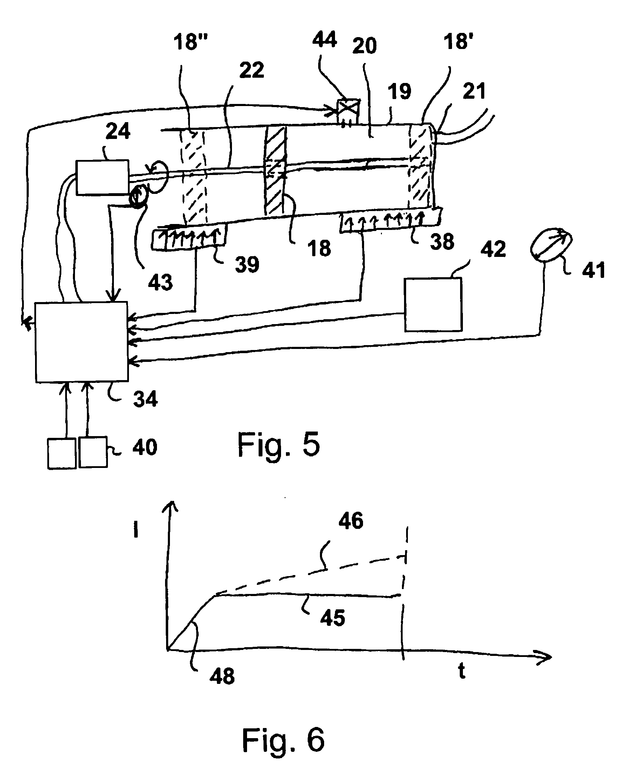

Dieser Mechanismus soll genauer anhand der Fig. 5 erläutert werden.

Fig. 5 zeigt schematisch mit wenigen Details die Arbeitspumpe mit dem Zylinder 19, dem Kolben 18, der Ausströmöffnung 21 und der durch den Motor 24 antreibbaren Spindel 22.

Der Kolben ist an einer zufällig ausgewählten Position dargestellt, jedoch sind zwei andere mögliche Positionen 18', 18 " gestrichelt eingezeichnet. Zudem sind Näherungssensoren 38, 39 in Form von Hall-Sensoren eingezeichnet, die registrieren, an welcher Stelle sich der Kolben 18 befindet, zumindest sofern er sich im Bereich eines seiner Umkehrpunkte befindet.

Die Steuerung 34 bekommt entweder über eine Eingabe 40 oder über die Synchronisation mit dem durch das Messgerät 41 laufend registrierten Blutdruck des Patienten eine Zykluszeit vorgegeben, in der der Kolben 18 eine vollständige Bewegungsperiode zurückgelegt haben muss. Eine Zeitmessungseinheit 42 gibt eine Zeitbasis an, wobei beispielsweise der Näherungsschalter 38 registriert, ob zum Ende der Zykluszeit der Kolben 18 seine Position 18' erreicht hat. Ist dies der Fall, bevor die Zykluszeit verstrichen ist, so wird für den nächsten Zyklus der Steuerstrom für den Motor 24 etwas abgesenkt. Erreicht der Kolben die Position 18' nicht rechtzeitig, so wird für den nächsten Zyklus die Stromstärke des Motors 24 etwas erhöht oder ein Stromstärkenprofil ausgewählt, das ein erhöhtes Strom/Zeitintegral liefert. Ein gegebenenfalls vorbestimmter Stromverlauf kann auch insgesamt angehoben oder abgesenkt werden.

Entsprechende Messungen können auch durch den Näherungsschalter 39 zum Ende der Saugphase ausgeführt werden. Gleichzeitig wird mittels des Drehzahlmessgerätes 43 laufend die Drehzahl der Spindel 22 gemessen. Bei dem gesteuerten Drehmoment des Motors 24 hängt die Drehzahl der Spindel 22 weitestgehend von dem Druck im Druckraum 20 der Arbeitspumpe ab, so dass aus der der Differenz der gemessenen Drehzahlen in der systolischen und diastolischen Phase zu bestimmten festgelegten Zykluszeiten die Abweichung des Ist-Druckes von dem mittleren Soll-Druck im Druckraum 20 bestimmt werden kann. Ist der Druck zu hoch, so wird im nächsten Zyklus zu Beginn der Kompressionsphase das Steuerventil 44 geöffnet oder länger als in dem vorangehenden Zyklus geöffnet. Damit kann das Arbeitsgas/Luft aus dem Druckraum 20 eine Zeitlang ausströmen, so dass der danach aufgebaute Druck etwas geringer bleibt als im vorangegangenen Zyklus.

Wenn der mittlere Ist-Druck zu niedrig / die Saugkraft zu groß ist, kann im nachfolgenden Zyklus zu Beginn der Saugphase das Ventil 44 eine Zeitlang geöffnet bleiben bzw. eine Zeitlang länger geöffnet werden als in den vorangegangenen Zyklen, um den erreichten Enddruck zu regeln.

Auf diese Weise werden Regelmechanismen verwendet, um einerseits die Drehzahl mittels der eingestellten Stromstärke zu regeln, darüber hinaus wird innerhalb eines Zyklus der mittlere Druck in der Saug- sowie in der Druckphase geregelt.

Benötigt die Arbeitspumpe mehr oder weniger Energie, da sie zu wenig oder zu viel Zeit für das Durchlaufen eines Zyklus benötigt, so wird die eingeprägte Stromstärke oder der eingeprägte Stromstärkeverlauf entsprechend den Erfordernissen nachgeregelt.

Figur 6 zeigt verschiedene Stromstärkenverläufe über die Zeit mit einer Rampe zu Beginn der Kolbenbewegung und einem konstanten 45 sowie einem linear ansteigenden 46 Stromverlauf danach. Entsprechende Kurven können auch in Form eines Stromstärke/Kolbenweg-Diagramms vorgegeben sein.

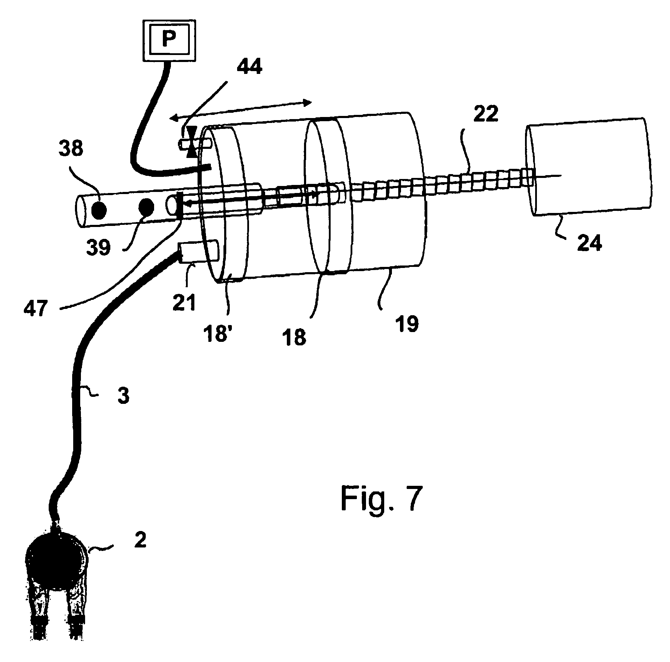

Figur 7 zeigt eine dreidimensionale Darstellung der Arbeitspumpe, bei der ein Steuerventil 44 an der Stirnseite des Zylinders 19, zwei magnetische Wegsensoren 38,39 am Führungsrohr 29 und ein Hall- Geber 47 an der Spindel 22 vorgesehen ist. Die Spindel 22 ist als Kugel- Gewindetrieb ausgebildet. Die Gewindemutter im Kolben 18 enthält entsprechend federnd gelagerte Führungskugeln.

Die Erfindung kommt insgesamt mit einem geringen Messaufwand aus, und besonders vorteilhaft können Flussmessungen und Volumenstrommessungen an dem System eingespart werden. Die Pumpe ist aufgrund der dynamischen Regelung in der Lage, in einem Förderbereich von pro Zyklus etwa 10 bis 80 Milliliter in der Membranpumpe zu arbeiten und dies bei einer Rate von bis zu 140- bis 150-mal pro Minute, so dass entsprechende Pumpen auch bei Kleinkindern ohne Probleme eingesetzt werden können.

In einer Ausführungsform kann die Erfindung folgendermaßen zusammengefasst werden: Der Motor einer pneumatischen Kolbenpumpe wird nach einem Geschwindigkeitsprofil angesteuert, gemäß dem der Arbeitskolben jeweils nach einer Beschleunigungsphase zunächst mit einer hohen Geschwindigkeit bewegt und danach zum Ende des vorgegebenen Weges verzögert wird.

Besonders einfach lässt sich ein solches Geschwindigkeitsprofil durch Ansteuerung eines Elektromotors der Pumpe mit einem vorgerechneten Konstantstrom verwirklichen.

Bei der Ansteuerung des Motors mit einem vorgerechneten Konstantstrom ergibt sich eine druckabhängige Drehzahl, die jeweils in Systole und Diastole in einem bestimmten Bezugspunkt gemessen wird und die die Eingangsgröße eines Reglers für die ventilgesteuerte Membranregelung der Blutpumpe ist, deren Stellgröße die Öffnungszeit eines Ventils am Zylinder ist.

## Patentansprüche

1. Verfahren zum Betrieb eines Pumpensystems (2,4) mit einer als Kolbenpumpe ausgebildeten Arbeitspumpe, die einen mittels eines Antriebsaggregats (24) oszillierend zwischen zwei Umkehrpunkten in einem Druckraum antreibbaren Arbeitskolben (18) aufweist, **dadurch gekennzeichnet, dass** die Kolbenpumpe derart angesteuert wird, dass der Antriebskolben (18) jeweils nach Durchlaufen eines Umkehrpunktes eine Beschleunigungsphase durchläuft und danach zum Ende des vorgegebenen Weges verzögert wird.

2. Verfahren zum Betrieb eines Herzpumpensystems (2,4)nach Anspruch 1 mit einer pulsatilen Membranblutpumpe (2) zum abwechselnden Ansaugen und Ausstoßen von Blut im Blutkreislauf eines Patienten, die mittels einer Druckleitung (3) mit der Arbeitspumpe (4) antreibbar ist, **dadurch gekennzeichnet, dass** jeweils nach Durchlaufen eines Umkehrpunktes vor Erreichen des Maximal-oder Minimaldrucks eine Geschwindigkeit des Arbeitskolbens (18) erreicht wird, die betragsmäßig größer, insbesondere wenigstens 10%, vorteilhaft mehr als 30%, weiter vorteilhaft mehr als 50% größer ist als die Geschwindigkeit bei Erreichen des Extremaldrucks.

3. Verfahren nach Anspruch 1 oder 2, **dadurch gekennzeichnet, dass** dem Arbeitskolben wenigstens in einem ersten, sich unmittelbar an einen Umkehrpunkt anschließenden Wegbereich ohne eine Drehzahlbegrenzung Energie zugeführt wird.

4. Verfahren nach Anspruch 3, **dadurch gekennzeichnet, dass** dem Arbeitskolben (18) wenigstens in dem ersten Wegbereich Energie durch das Antriebsaggregat und zusätzlich die in dem Druckraum vor Erreichen des Umkehrpunktes durch den erzeugten Unter- oder Überdruck gespeicherte Energie zugeführt wird.

5. Verfahren nach Anspruch 1,2,3 oder 4 **dadurch gekennzeichnet, dass** das Antriebsaggregat(24) bezüglich seines Drehmoments und/oder seiner Leistung als Zielgröße gesteuert wird.

6. Verfahren nach Anspruch loder einem der folgenden, **dadurch gekennzeichnet, dass** das Antriebsaggregat als Elektromotor ausgebildet ist und der Elektromotor (24) mittels eines eingeprägten, für wenigstens einen Arbeitshub, insbesondere für einen Arbeitszyklus unter Berücksichtigung einer Sollrate und/oder eines Sollhubes des Arbeitskolbens vorherbestimmten Stromstärkenverlaufs angesteuert wird.

7. Verfahren nach Anspruch 6, **dadurch gekennzeichnet, dass** bei dem jeweils zu ermittelnden Stromstärkenverlauf Messwerte, aus denen Abweichungen der Ist- Werte vom Sollhub und der Sollrate ermittelt werden können, oder die ermittelten Abweichungen berücksichtigt werden.

8. Verfahren nach Anspruch 5,6 oder 7, **dadurch gekennzeichnet, dass** das Antriebsaggregat als Elektromotor ausgebildet ist und der Elektromotor(24) mittels eines zumindest zeitweise linearen Stromstärkenverlaufs angesteuert wird.

9. Verfahren nach Anspruch 8, **dadurch gekennzeichnet, dass** der Stromstärkenverlauf in mindestens einer der beiden zyklisch wiederkehrenden Bewegungsphasen des Kolbens eine lineare Anstiegsrampe (48) aufweist.

10. Verfahren nach Anspruch 9, **dadurch gekennzeichnet, dass** nach der Anstiegsrampe des Stromstärkenverlaufs die Stromstärke entweder bis zum nächsten Umkehrpunkt des Kolbens (18) konstant bleibt oder linear ansteigt.

11. Verfahren nach Anspruch 1 oder einem der folgenden, **dadurch gekennzeichnet, dass** ein Steuerventil (44), das einen Druckraum (20) der Arbeitspumpe (4) mit einem Ausgleichsvolumen verbindet, zyklisch in Abhängigkeit von ermittelten Abweichungen der gemessenen Bewegungsgeschwindigkeiten des Arbeitskolbens oder Drehzahlen des Antriebsaggregats oder hieraus abgeleiteter Druckwerte von vorgegebenen Referenzwerten angesteuert wird.

12. Verfahren nach Anspruch 11, **dadurch gekennzeichnet, dass** in Abhängigkeit von den Abweichungen die Öffnungsdauer des Steuerventils (44) jeweils in jeder Druckphase und/oder der Saugphase, insbesondere jeweils am Anfang der jeweiligen Phase, vorgegeben wird.

13. Verfahren nach Anspruch 1 oder einem der folgenden, **dadurch gekennzeichnet, dass** die Bewegungsgeschwindigkeit des Antriebsaggregats (24) und/oder des Kolbens (18), insbesondere die Drehzahl des Motors, zyklisch wenigstens einmal zu einem Zykluszeitpunkt während jeder Druck-und jeder Saugphase (Systole und Diastole) gemessen wird, zu dem die gemessene Größe im Wesentlichen von dem durch die Arbeitspumpe erzeugten Druck abhängt, und dass aus dem Vergleich dieser Messwerte miteinander Öffnungsdauern eines Steuerventils zwischen dem Druckraum der Arbeitspumpe und einem Druckausgleichsraum sowohl für die Druckphase als auch für die Saugphase ermittelt werden.

14. Verfahren nach Anspruch 12 oder 13, **dadurch gekennzeichnet, dass** jeweils die Maximaldrehzahlen des Motors (24) in der Druck- und in der Saugphase bestimmt und miteinander zur Ermittlung von Abweichungen verglichen werden.

15. Verfahren nach Anspruch 11, 12 oder 13, **dadurch gekennzeichnet, dass** aus den beiden Drehzahlen, die in Systole und Diastole jeweils zu dem Zeitpunkt gemessen werden, wenn der Druck den mittleren Systemdruck erreicht hat, insbesondere aus der Differenz dieser beiden Drehzahlen, die Steuergröße für ein Steuerventil (44) berechnet wird, das den Druckraum (20) der Arbeitspumpe (4) für eine steuerbare Zeit während der Systole und der Diastole, insbesondere jeweils nach dem Durchlaufen des Drucknullwertes, mit einem Ausgleichsvolumen verbindet.

16. Verfahren nach Anspruch 5 oder einem der folgenden, **dadurch gekennzeichnet, dass** ein Abbremsen der Arbeitskolbenbewegung darauf untersucht wird, an welcher Position des Kolbens (18) diese auftritt und/ oder wie stark die Bremsbeschleunigung ist und dass bei einer bestimmten Abweichung vom Normalverhalten auf Vorliegen einer Blockade der Blutkanülen oder der Druckleitung erkannt wird.

17. Pumpensystem, betreibbar mit einem Verfahren gemäß einem der Ansprüche 1 bis 16, mit einer Membranblutpumpe (2), die durch eine Arbeitspumpe (4) mittels einer Druckleitung (3) antreibbar ist, wobei die Arbeitspumpe einen mittels eines Elektromotors (24) oszillatorisch antreibbaren Kolben (18) in einem Zylinder (19) aufweist, **gekennzeichnet durch** ein mittels einer Steuerung ansteuerbares Steuerventil(44), das einen Druckraum (20) der Arbeitspumpe (4) mit einem Ausgleichsvolumen verbindet

18. Verfahren nach Anspruch 1,2,3 oder 4, **dadurch gekennzeichnet, dass** der Kolben in der Druckphase bis dicht an das Zylinderende bewegt wird, wobei ein Bezugspunkt bei Kolbenstellung nahe am Zylinderende gleichzeitig als Zielpunkt für den Systolenendpunkt und den Diastolenanfangspunkt definiert wird.
